# EUROPEAN PATENT SPECIFICATION

(11) **EP 3 720 548 B1**
(45) Date of publication and mention of the grant of the patent: **01.11.2023**
(21) Application number: 18885503.5
(22) Date of filing: 03.12.2018
(51) Int. Cl.: A61N 1/36, A61N 1/04, A61N 1/05

(54) **ELICITING SWALLOWING USING ELECTRICAL STIMULATION APPLIED VIA SURFACE ELECTRODES**
AUSLÖSEN DES SCHLUCKENS UNTER VERWENDUNG EINER ÜBER OBERFLÄCHENELEKTRODEN AUFGEBRACHTEN ELEKTRISCHEN STIMULATION
DÉCLENCHEMENT DE LA DÉGLUTITION À L'AIDE D'UNE STIMULATION ÉLECTRIQUE APPLIQUÉE PAR L'INTERMÉDIAIRE D'ÉLECTRODES DE SURFACE

(30) Priority: 04.12.2017 US 201762594109 P
(43) Date of publication of application: 14.10.2020
(73) Proprietor: MED-EL Elektromedizinische Geraete GmbH, 6020 Innsbruck (AT)
(72) Inventor: LINDENTHALER, Werner, A-6173 Oberperfuss (AT); LADURNER, Matthias, 6152 Trins (AT); MAYR, Winfried, A-2340 Mödling (AT); KRENN, Matthias, Jackson, MS 39202-1319 (US)
(74) Representative: Downing, Michael Philip
(86) International application number: PCT/US2018/063567
(87) International publication number: WO 2019/112935

(56) References cited:
- WO-A1-2014/036425
- WO-A1-2017/002901
- WO-A1-2017/025720
- US-A1- 2002 133 194
- US-A1- 2007 123 950
- US-A1- 2010 241 191
- US-A1- 2015 164 737
- US-A1- 2015 165 201
- US-B2- 6 484 053
- US-B2- 7 039 468
- US-B2- 8 583 240
- US-B2- 8 965 535
- US-B2- 9 440 075
- CUMMINGS JP: "Conservative management of peripheral nerve injuries utilizing selective electrical stimulation of denervated muscle with exponentially progressive - - current forms", J Orthop Sports Phys Ther., vol. 7, no. 1, 1985, pages 11-15, XP055616977,
- SCHUHFRIED O et al.: "Reliability of chronaxie and accommodation index in the diagnosis of muscle denervation", Physikalische Medizin, Rehabilitationsmedizin, Kurortmedizin (Phys Med Rehab Kuror), vol. 15, no. 3 2005, pages 174-178, XP009520508, DOI: 10.1055/s-2004-834713 Retrieved from the Internet: URL:https://www.thieme-connect.de/media/ph ysmed/200503/lookinside/10.1055-s-2004-834 713-1.jpg [retrieved on 2019-01-24]

## Description

### Technical Field

The present invention relates to a system for eliciting swallowing; and more particularly, to elicit swallowing using electrical stimulation applied via surface electrodes.

### Background Art

Swallowing is a vital and complicated process that involves numerous muscles. Stroke, neurodegenerative diseases, brain tumors, respiratory disorders, and the like may cause dysphagia resulting in insufficient control of those muscles needed for swallowing. In severe cases, aspiration pneumonia may result. Dysphagia often results from poor control of some muscles in the upper respiratory system. Many muscles in this system are also involved with speech and voice.

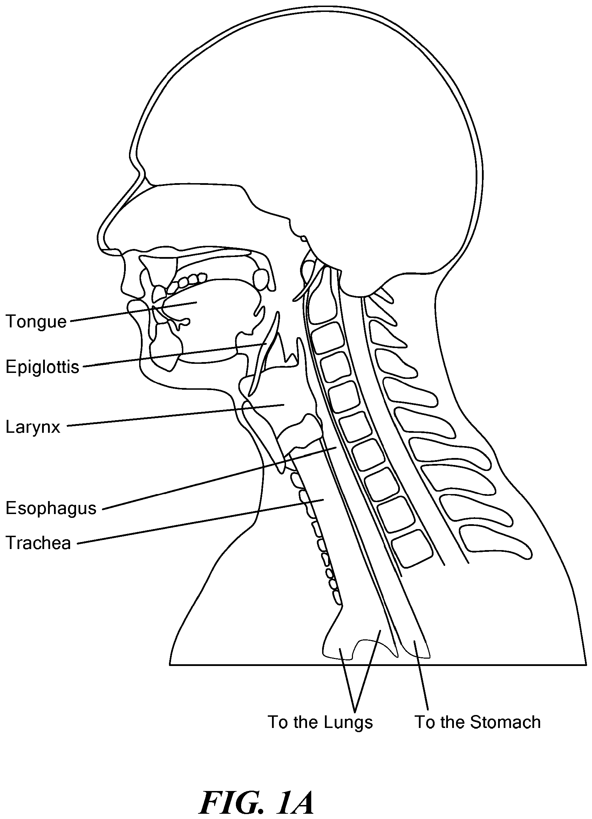

Fig. 1A shows various structures of the neck and mouth that are involved in swallowing. In general, swallowing disorders are predominately a human problem because the ability to have highly developed speech is directly related to the much lower location of the larynx (lower compared to the larynx of many animals) which in turn enhances the dangers associated with swallowing. The larynx is located in the neck and is involved in breathing, operation of voice and protecting the trachea from aspiration of food and water via the esophagus. The complete closure of the larynx and its timing is critical to ensure safe swallowing, as it controls whether food enters the trachea or the esophagus. In case the larynx fails to close, food or liquids may detrimentally enter the airway. The higher position of the larynx in some animals, in which the larynx is not a highly developed natural speech apparatus, even allows these animals to drink and breathe simultaneously-something which is impossible for human beings.

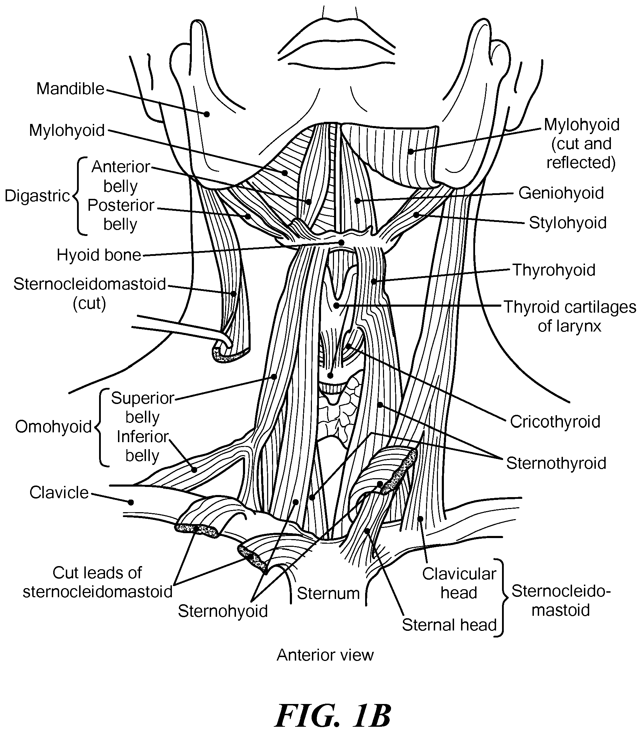

In humans, at least twelve muscles are involved in the swallowing process. Fig. 1B is an anterior view of a human's neck showing various muscles and associated structures in their natural positions. Proper control of their movement is particularly important since the failure of movement may have critical consequences. However, there is no clear understanding of which muscles may predominate or even if proper swallowing requires coordinated contraction of all twelve or more muscles since the swallowing process varies from person to person. For example, the geniohyoid, mylohyoid and digastric muscles are used selectively by different individuals, e.g., some use all three muscles at the onset of swallowing, while others use different pairs. In addition, the temporal association between submental muscle contractions differs across individuals.

Electrical stimulation has been successfully used for controlling weakened muscles/nerves, such as aged or degenerated nerves/muscles, for controlling re-innervating nerves, including synkinetically re-innervating nerves, and/or for providing electrical signals to nerves in order to compensate for hearing deficiencies (e.g., cochlear implant stimulation for providing hearing sensations to deaf people) or to overrule wrong elicited nerve signals.

While various stimulation systems and methods have been proposed to control the upper respiratory muscles used for swallowing, none of them address the elevation of the larynx in order to provide for proper swallowing. For example, Freed et al. describe a non-invasive method and apparatus that continuously stimulates the skin surface to assist patients in initiating a swallow (see, e.g., U.S. Pat. Nos. 5,725,564, 6,104,958, and 5,891,185). In addition, there are systems which cause glottis closure by means of appropriate electrical stimulation (see, e.g., Bidus et al., Laryngoscope, 110:1943-1949, 2000; Ludlow et al., Journal of Artificial Organs, 23:463-465, 1999; and Ludlow et al., Muscle and Nerve, 23:44-57, 2000). In U.S. Pat. Appl. No. 2007/0123950, Ludlow et al. (hereinafter Ludlow '950) disclose a method and system for synergistic production of muscle movements during speech, swallowing or voice production by moving the hyoid bone and/or parts of the upper airway and/or vocal tract by means of electrical stimulation of at least two different muscles using implanted electrodes. Ludlow '950 found that neuromuscular stimulation of only two of the muscles yields a large proportion of normal desired movement for the hyoid bone. Further, Ludlow discloses that the muscles involved in swallowing remain at their normal, given locations within a human's body. The reader is referred to the above cited documents for a fuller understanding.

Using conventional techniques, eliciting swallowing using surface electrodes remains problematic since pain thresholds are often exceeded or unwanted responses are activated. Some approaches include inserting a catheter into the pharynx/esophagus through the nose applying electrical stimulation impulses onto electrodes located on the catheter. This is painful and unpleasant for the patient and is not a long term solution.

Another approach using surface electrodes to elicit swallowing is by applying regular, short (e.g., 0.2ms), rectangular pulses. Although surface electrical stimulation has received increased attention as an adjunct to swallowing therapy in dysphagia in recent years (see, for example: Freed ML, et al. Electrical stimulation for swallowing disorders caused by stroke. Respir Care. 2001;46(5):466-74. [PubMed]; Leelamanit V, Limsakul C, Geater A. Synchronized electrical stimulation in treating pharyngeal dysphagia. Laryngoscope. 2002;112(12):2204-10. [PubMed]; Park CL, O'Neill PA, Martin DF. A pilot exploratory study of oral electrical stimulation on swallow function following stroke: an innovative technique. Dysphagia. 1997;12(3):161-6. [PubMed]; and Power M, et al. Changes in pharyngeal corticobulbar excitability and swallowing behavior after oral stimulation. Am J Physiol Gastrointest Liver Physiol. 2004;286(1):G45-50. [PubMed], to which the reader is referred for a fuller understanding), little is known about the effects of transcutaneous stimulation on swallowing physiology before the study of Christy L. Ludlow, Ianessa Humbert, Keith Saxon, Christopher Poletto, Barbara Sonies, and Lisa Crujido. Effects of Surface Electrical Stimulation Both at Rest and During Swallowing in Chronic Pharyngeal Dysphagia. Dysphagia. 2007 Jan; 22(1): 1-10, to which the reader is referred for a fuller understanding. It had been hypothesized that electrical stimulation may assist swallowing either by augmenting hyo-laryngeal elevation (See, for example, Freed et al. *Electrical stimulation for swallowing disorders caused by stroke.,* and Leelamanit et al.) or by increasing sensory input to the central nervous system to enhance the elicitation of swallowing (See, for example, Park et al., and Power et al.)

As described in Ludlow et al. *Effects of Surface Electrical Stimulation Both at Rest and During Swallowing in Chronic Pharyngeal Dysphagia,* electrical stimulation applied to the skin at low current levels activates sensory nerve endings in the surface layers providing sensory feedback to the central nervous system. When the amplitude is increased, the electric field may depolarize nerve endings in muscles lying beneath the skin surface (see Loeb GE, Gans C. Electromyography for Experimentalists. The University of Chicago; Chicago: 1986, to which the reader is referred for a fuller understanding) and may spread with diminishing density to produce muscle contraction. Accordingly, when electrodes are placed in the submental region of the neck, current density is greatest at the skin surface, and diminishes with depth through the platysma underlying the skin and subcutaneous fat (See Sobotta J. Sobotta Atlas of Human Anatomy. In: Staubesand J, editor. Head, Neck, Upper limbs, skin. 11th English Edition ed Volume 1. Urban & Schwarzenberg; Baltimore --Munich: 1990, to which the reader is referred for a fuller understanding). If the current is increased in amplitude, increasingly deeper muscles may be recruited, albeit with less efficiency. Such muscles may include the anterior belly of the digastric, which can either lower the mandible or pull the hyoid upward depending upon whether the mouth is held closed. Deeper still are the mylohyoid and geniohyoid muscles, which pull the hyoid bone upward and toward the mandible, respectively. These muscles are much less likely to be activated by surface stimulation, however, because of their greater depth.

Similarly, when electrodes are placed on the skin overlying the thyroid cartilage in the neck, the current will be greatest at the skin with less intensity to the underlying platysma muscle, and even less intensity to the underlying sternohyoid and omohyoid muscles (See Sobatta), which pull the hyoid downward and backward towards the sternum. The electrical field strength is even further diminished upon reaching: the deeper thyrohyoid muscle, which brings the larynx and hyoid together; and the sternothyroid muscle, which lowers the larynx towards the sternum. Given that the sternohyoid muscle is larger and overlies the thyrohyoid and sternothyroid, the high levels of surface electrical stimulation on the neck most likely would pull the hyoid downward due to stimulation of either the sternohyoid or the underlying sternothyroid, but would be much less likely to raise the larynx toward the hyoid bone as occurs in normal swallowing.

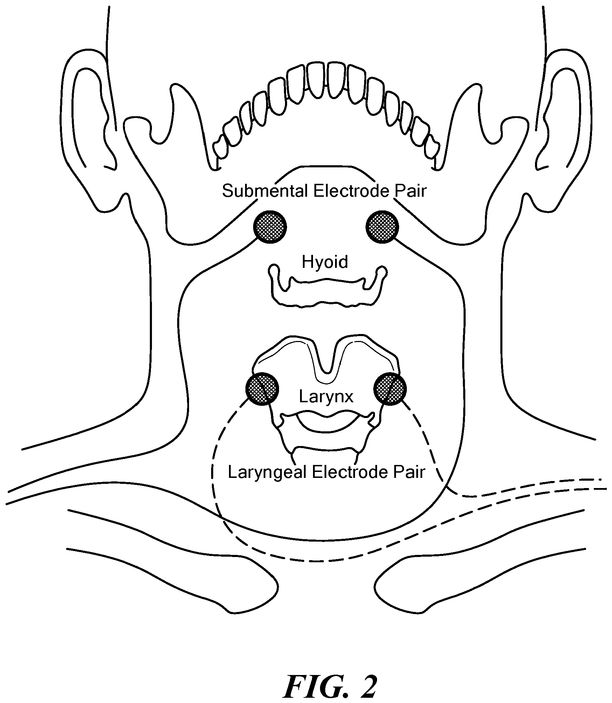

In Ludlow et al. *Effects of Surface Electrical Stimulation Both at Rest and During Swallowing in Chronic Pharyngeal Dysphagia,* both submental and laryngeal pairs of electrodes were used simultaneously, as recommended for VitalStim^{®} Therapy (See Wijting Y, Freed ML. VitalStim Therapy Training Manual. Chattanooga Group; Hixson, TN: 2003, to which the reader is referred for a fuller understanding), with the aim of producing a simultaneous contraction of the mylohyoid in the submental region (to elevate the hyoid bone) and the thyrohyoid in the neck (to elevate the larynx to the hyoid bone). Two sets of electrodes were used, the top set was placed horizontally in the submental region over the region of the mylohyoid muscle above the hyoid bone, as shown in Fig. 2. However, because these muscles lie deep beneath the anterior belly of the digastric, sternohyoid and omohyoid muscles, Ludlow et al. *Effects of Surface Electrical Stimulation Both at Rest and During Swallowing in Chronic Pharyngeal Dysphagia* hypothesized that simultaneous transcutaneous stimulation with two pairs of electrodes at rest would cause: 1) the hyoid bone to descend in the neck (due to sternohyoid muscle action); 2) the hyoid bone to move posteriorly (due to the omohyoid muscle activity); and, 3) the larynx to descend (if current activates either the sternohyoid or stenothyroid muscles). Further, in severe chronic dysphagia: 4) when the same array is used at low levels of stimulation just above the sensory threshold, sufficient for sensation but without muscle activation, patients' swallowing might improve due to sensory facilitation; while 5) at higher levels required for motor stimulation, the descent of the hyoid might interfere with swallowing causing increased penetration and aspiration.

The results in Ludlow et al. *Effects of Surface Electrical Stimulation Both at Rest and During Swallowing in Chronic Pharyngeal Dysphagia* did indicate that when surface stimulation was applied to the neck at rest, stimulation was either too weak or not deep enough to stimulate axons innervating the muscles that produce hyoid and laryngeal elevation such as the mylohyoid and the thyrohyoid muscles, respectively. No change in laryngeal position was observed with surface stimulation at rest. Those patients who did have reduced aspiration and penetration during swallowing with stimulation had greater hyoid depression during stimulation at rest. When the hyoid was depressed with stimulation, Ludlow et al. *Effects of Surface Electrical Stimulation Both at Rest and During Swallowing in Chronic Pharyngeal Dysphagia* conjectured that a patient probably experienced a greater resistance to hyo-laryngeal elevation during swallowing. Perhaps those patients who felt a greater downward pull on the hyoid, when stimulation was turned on at maximal levels, made a greater effort to elevate the hyo-laryngeal complex when swallowing in an attempt to overcome the effects of the stimulation. It could also be the case that those patients who had greater residual power in their hyo-laryngeal muscles would have not only experienced greater hyoid descent with stimulation but could also have greater residual power that they could recruit for hyo-laryngeal elevation to counteract the stimulation induced descent during swallowing.

As Ludlow et al. *Effects of Surface Electrical Stimulation Both at Rest and During Swallowing in Chronic Pharyngeal Dysphagia* theorized, it is likely that the simultaneous stimulation resulted in hyoid lowering because the stronger stimulation to the more superficial and larger sternohyoid and sternothyroid muscles overcame any action that might have been induced by stimulation of the mylohyoid muscle in the submental region or the thyrohyoid muscle beneath the sternohyoid in the throat region. Some have proposed using submental stimulation alone to activate the anterior belly of the digastric and the mylohyoid muscles to pull the hyoid bone upward. However, elevation of the hyoid bone without simultaneous stimulation of the thyrohyoid to raise the larynx would leave the larynx down resulting in further opening of the vestibule and increased risk of aspiration. Only if the mylohyoid and thyrohyoid muscles are activated together, without contraction of the sternohyoid, would both the hyoid and larynx be raised together as has previously been shown with intramuscular stimulation (See Burnett TA, et al. Laryngeal elevation achieved by neuromuscular stimulation at rest. J Appl Physiol. 2003;94(1):128-34, to which the reader is referred for a fuller understanding). This is problematic using surface stimulation, because the larger sternohyoid muscle overlies the thyrohyoid and pulls the hyoid downward.

Other documents of potential interest include US7039468, US2010241191, WO2014036425, US2015165201, WO2017025720, US2002133194, US6484053, US2007123950, US8583240, US8965535, US2015164737, US9440075, WO2017002901, CUMMINGS JP, "Conservative management of peripheral nerve injuries utilizing selective electrical stimulation of denervated muscle with exponentially progressive - - current forms", J Orthop Sports Phys Ther., (19850000), vol. 7, no. 1, pages 11 - 15, XP055616977 [Y] 3-4, 15-16, and SCHUHFRIED O et al., "Reliability of chronaxie and accommodation index in the diagnosis of muscle denervation", Physikalische Medizin, Rehabilitationsmedizin, Kurortmedizin (Phys Med Rehab Kuror), vol. 15, no. 3, doi:10.1055/s-2004-834713, (20050000), pages 174 - 178 which disclose ways of controlling or assisting with swallowing, and related topics.

### Summary of the Embodiments

In accordance with an embodiment of the invention, a system for eliciting a full swallowing reflex in a human subject is provided, as set out in claim 1. The system includes a plurality of surface electrodes configured to overlay at least a region of thyroid cartilage in the neck. A controller is configured to generate a stimulation signal for eliciting the full swallowing reflex in the human subject, the controller configured to provide the stimulation signal to the plurality of surface electrodes upon receipt of a trigger signal.

The controller is configured to generate a bipolar stimulation pulse having a first pulse phase duration of at least 100ms. The first phase of the bipolar stimulation pulse may have an increasing magnitude over time. The first pulse phase of the bipolar stimulation signal may be one of progressively rising, exponentially rising, linearly rising, curvilinearly rising, continuously rising or discontinuously rising, and combinations thereof. The surface electrodes may include a first surface electrode and a second surface electrode in a bipolar configuration, the first surface electrode and the second surface electrode for positioning on the skin to the left and right of the laryngeal prominence, respectively, so as to overlay the region of thyroid cartilage in the neck.

In accordance with further related embodiments of the invention, the controller may be configured to generate a unipolar stimulation pulse. The unipolar stimulation pulse may be a triangular pulse that includes a rising edge with a duration of at least 100ms. The surface electrodes may include a first surface electrode and a second surface electrode for positioning on the skin to the left and right of the laryngeal prominence, respectively, so as to overlay the region of thyroid cartilage in the neck, and a third surface electrode and a fourth surface electrode for placing on left and right shoulder blades of the human subject.

The system may include a trigger mechanism configured to provide the trigger signal. The trigger mechanism may include one of a hand switch for activating by the human subject, a movement sensor, an inertial mass sensor, a sensor for electromyography of the submental muscles, or a sensor for measuring bioimpedances of the neck, and combinations thereof. The swallowing reflex may be elicited in human subjects who do suffer from denervated laryngeal muscles and in subjects who do not suffer from denervated laryngeal muscles.

In accordance with yet further embodiments of the invention, a system for eliciting a full swallowing reflex in a human subject is provided, as set out in claim 10. The system includes a plurality of electrodes configured to be implanted adjacent at least thyroid cartilage in the neck. A controller is configured to generate a stimulation signal for eliciting the full swallowing reflex in the human subject, the controller configured to provide the stimulation signal to the plurality of electrodes upon receipt of a trigger signal.

In accordance with related embodiments of the invention, the controller may be configured to generate a bipolar stimulation pulse having a first pulse phase duration of at least 100ms. The first phase of the bipolar stimulation pulse may have an increasing magnitude over time. The first pulse phase of the bipolar stimulation signal may be one of progressively rising, exponentially rising, linearly rising, curvilinearly rising, continuously rising or discontinuously rising, and combinations thereof. The electrodes may include a first electrode and a second electrode in a bipolar configuration.

In accordance with further related embodiments of the invention, the controller may be configured to generate a unipolar stimulation pulse. The unipolar stimulation pulse may be a triangular pulse that includes a rising edge with a duration of at least 100ms.

The system may include a trigger mechanism configured to provide the trigger signal. The trigger mechanism may include one of a hand switch for activating by the human subject, a movement sensor, an inertial mass sensor, a sensor for electromyography of the submental muscles, or a sensor for measuring bioimpedances of the neck, and combinations thereof. The swallowing reflex may be elicited in human subjects who do suffer from denervated laryngeal muscles and in subjects who do not suffer from denervated laryngeal muscles.

### Brief Description of the Drawings

The foregoing features of embodiments will be more readily understood by reference to the following detailed description, taken with reference to the accompanying drawings, in which:
Fig. 1A shows various structures of the neck and mouth that are involved in swallowing;
Fig. 1B is an anterior view of a human's neck showing various muscles and associated structures in their natural positions;
Fig. 2 shows a prior art electrode configuration using to treat dysphagia;
Fig. 3 shows a system for eliciting a full swallowing reflex, in accordance with an embodiment of the invention;
Fig. 4 shows a bipolar electrode configuration, in accordance with an embodiment of the invention;
Fig. 5 shows a unipolar electrode configuration, in accordance with an embodiment of the invention;
Fig. 6 shows rheobase and chronaxie points defined on a strength-duration curve for stimulus of an excitable tissue;
Fig. 7 shows a strength-duration curve of a normally innervated muscle and a denervated muscle;
Fig. 8 shows different accommodation indices in human muscle groups;
Fig. 9 shows grading of muscle denervation according to electrical muscle testing and EMG; and
Fig. 10 shows the principle of selective stimulation of denervated muscle utilizing a long exponentially progressive current form.

### Detailed Description of Specific Embodiments

In illustrative embodiments, a system and method of eliciting a full swallowing reflex in a human subject is provided. More particularly, illustrative embodiments of the invention are based on the surprising and completely unexpected finding that long, slowly rising stimulation pulses elicit a full swallowing reflex in human subjects. Such stimulation pulses are usually only used for persons with denervated muscles. Details hereto are described below. This swallowing reflex may be elicited in human subjects who do suffer from denervated laryngeal muscles and in subjects who do not suffer from denervated laryngeal muscles.

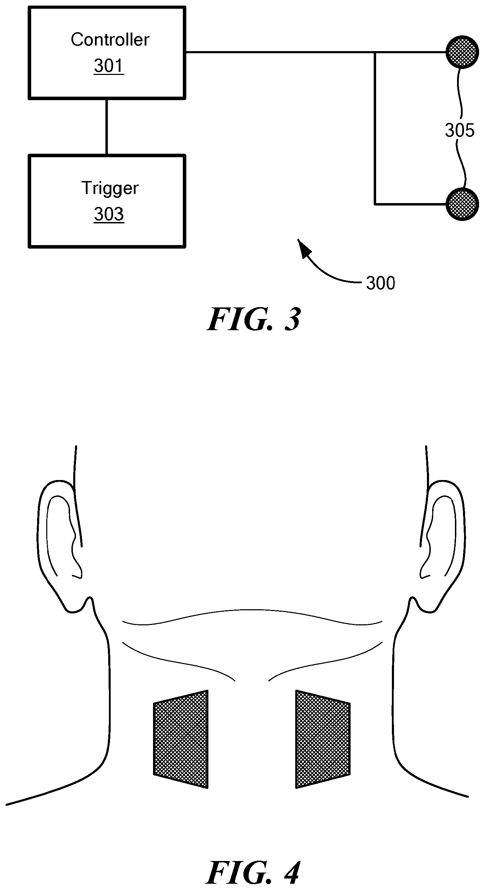

Fig. 3 shows a system 300 for eliciting a full swallowing reflex, in accordance with an embodiment of the invention. The full swallowing reflex not only activates single muscles in the deep neck region only, but includes activating full swallowing cycles which include coordinated contraction of, for example, twelve or more muscle pairs with appropriate timing and peristaltic manner and/or activation of one or more players of the afferent leg of the swallowing circuit. The full swallowing reflex advantageously includes raising of the larynx.

The complex pharyngeal phase of swallowing begins with the triggering of the swallowing reflex and ends with the opening of the upper esophageal sphincter and takes 0.7 to 1 second. It is not voluntarily controllable. During this phase the pharyngeal space expands for bolus passage, pressure is built up to promote bolus transportation, and the airways are closed to protect against aspiration. Rapid piston-like movements of the tongue support passage of the bolus into the hypopharynx. Peristaltic movements of the pharyngeal wall support the piston function of the tongue. Depending on the bolus volume, hyoid bone and the larynx move upwardly due to contraction of the suprahyoid muscles. This motion results in an expansion of space in the hypopharynx, positioning of the larynx under the root of the tongue to prevent aspiration, improved epiglottic tilting, and opening of the pharyngo-esophageal segment. To protect against aspiration, closure of the larynx proceeds in 3 stages: closure of the vocal folds, vertical approach of the adducted arytenoids to the base of the epiglottis, and epiglottic tilting to cover the laryngeal vestibule. Closure of the epiglottis is made possible by the bolus pressure from above, the downward muscular action of the ary epiglottic muscles, and the combined pressure as a result of the backward movement of the tongue and the laryngeal elevation. Opening of the upper esophageal sphincter is made possible by the anterior-superior movement of hyoid bone and larynx. The pharyngeal phase ends as soon as the bolus has reached the upper esophageal sphincter. Thereafter, the pharyngo-esophageal element, the tongue, hyoid and larynx return to their original positions. Velopharyngeal and laryngeal closures open up, and the pharyngo-esophageal element is closed.

The esophageal phase begins with the closure of the pharyngo-esophageal segment and lasts for 8 to 20 s. Bolus transportation proceeds by means of primary peristaltic waves induced by the swallowing reflex and, secondarily, by local stretch stimuli.

Sensory involvement in the swallowing is described in: Pommerenke WT. A study of the sensory areas eliciting the swallowing reflex. American Journal of Physiology. 1927;84(1):36-41; Jean A. Control of the central swallowing program by inputs from the peripheral receptors. A review. J Auton.Nerv Syst. 1984;10:225-233. [PubMed]; Jafari S, et al. Sensory regulation of swallowing and airway protection: a role for the internal superior laryngeal nerve in humans. J Physiol. 2003;550(Pt 1):287-304; and Hamdy S, et al. Modulation of human swallowing behaviour by thermal and chemical stimulation in health and after brain injury. Neurogastroenterol Motil. 2003;15(1):69-77. [PubMed], The reader is referred to each of the above-described references for a fuller understanding.

Referring back to Fig. 3, the system 300 may include, without limitation, a plurality of surface electrodes 305 configured to overlay at least thyroid cartilage in the neck. A controller 301 is configured to generate a stimulation signal for eliciting the full swallowing reflex in the human subject. The controller 301 is further configured to provide the stimulation signal to the plurality of surface electrodes 305 upon receipt of a trigger signal. The controller 301 may include, without limitation, a circuit and/or a processor that may be pre-programmed or configured to be loaded with an appropriate software program to generate and provide the stimulation signal.

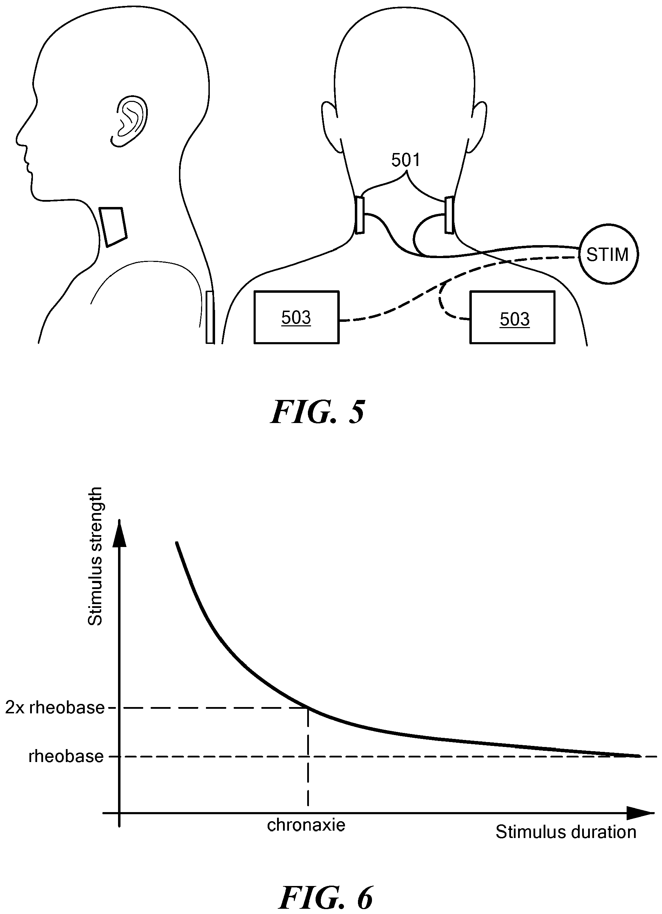

In various embodiments, the electrode stimulation set-up may be a bipolar electrode configuration. Illustratively, as shown in Fig. 4, the electrodes 401 may be placed left and right of laryngeal prominence, i.e., they are placed on the skin overlying the thyroid cartilage in the neck. In alternative embodiments, the electrode stimulation set-up may be unipolar electrode configuration. Illustratively, as shown in Fig. 5, two electrodes (similar to the bipolar set-up) 501 may be placed above the larynx, i.e. on the skin overlying the thyroid cartilage in the neck, and two indifferent electrodes 503 on the shoulder blades (for, example, large self-adhesive electrodes with 13 x 8 cm), in accordance with an embodiment of the invention.

With conventional stimulation systems and methodologies, stimulation of muscle and/or nervous tissue with bipolar pulses of typically .05 to 50 ms duration using electrodes placed on the skin overlying the thyroid cartilage in the neck caused stimulation of the underlying platysma muscle and the underlying sternohyoid and omohyoid muscles (see Sobotta). This in turn pulled the hyoid downward and backward towards the sternum and the sternothyroid muscle, which lowers the larynx towards the sternum, which could pull the hyoid downward due to stimulation of either the sternohyoid or the underlying sternothyroid, but would be much less likely to raise the larynx toward the hyoid bone as occurs in normal swallowing. In some circumstance such stimulation may also cause pain.

In order to avoid the drawbacks of these conventional systems and methodologies, embodiments of the invention may take advantage of the accommodation principle by using very long phase duration (greater than 100 ms) combined with slowly rising phase shapes (pulses similar to those used in various treatments of denervated muscle tissue). Accommodation is the muscle capacity not to respond to slowly incrementing electrical pulses. Accommodation describes the response of excitable membranes to slow depolarizing currents without the generation of action potential.

In order to describe accommodation, first the terms rheobase and chronaxie are explained with the help of strength-duration curves. Strength-duration curves are graphic representations of the relationship between the intensity of an electric stimulus and the length of time it must flow to elicit a minimal contraction (See, for example, Rodríguez-Fernández, Á. L., Rebollo-Roldán, J., Jiménez-Rejano, J. J., & Güeita-Rodríguez, J. (2016). Strength-duration curves of the common fibular nerve show hypoexcitability in people with functional ankle instability. PM and R, pp. 536-544, to which the reader is referred for a fuller understanding) In functional electrical stimulation, the strength-duration curve is often useful in determining characteristics of a stimulation electrode and determining the most efficient selection of stimulation parameters for an appropriate safety margin.

Fig. 6 shows rheobase and chronaxie points defined on a strength-duration curve for stimulus of an excitable tissue. Usually around the 1 ms mark on the strength-duration curve, the curve flattens out at the Rheobase, this is the point where a progressive increase in pulse duration is no longer associated with a progressive decrease in voltage. In other words, for longer stimulus durations, the minimal voltage required to bring the nerve to threshold will be the Rheobase. Given that two nerves have the same Rheobase, the chronaxie (the stimulus duration corresponding to twice the rheobase) can give an indication of their relative excitabilities. The smaller the Chronaxie the more excitable the nerve is.

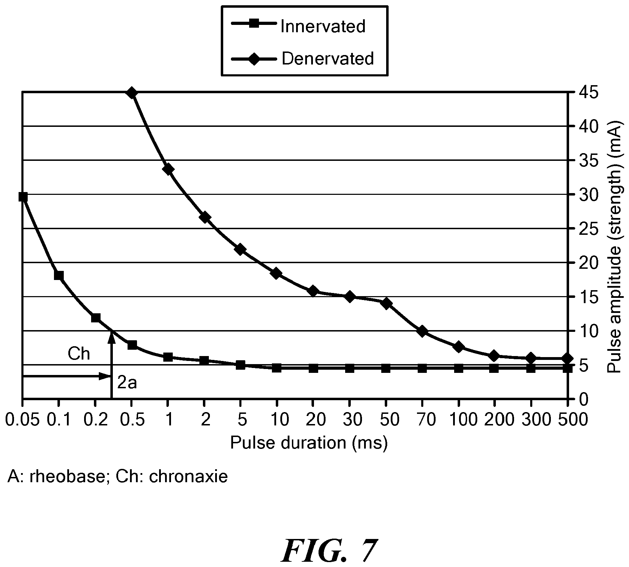

Fig. 7 shows a strength-duration curve of a normally innervated muscle and a denervated muscle, as depicted in Schuhfried, O., Kollmann, C., & Paternostro-Sluga, T. (2005). Excitability of chronic hemiparetic muscles: determination of chronaxie values and strength-duration curves and its implication in functional electrical stimulation. IEEE Transactions on Neural Systems and Rehabilitation Engineering : A Publication of the IEEE Engineering in Medicine and Biology Society, 13(1), 105-9), to which the reader is referred for a fuller understanding. The average normal chronaxie value of an innervated muscle is 0.4 ms (see Schuhfried et al.). Chronaxie values above 1 ms are considered as evidence of muscle denervation. The shift to the right and the appearance of kinks in the strength-duration curves is associated with muscle denervation. The strength-duration curve was determined by successively decreasing the widths of monophasic rectangular impulses (500, 300, 200, 100, 70, 50, 30, 20, 10, 5, 2, 1, 0.5, 0.2, 0.1, 0.05 ms).

The Chronaxie values for human arm sensory nerves range from 0.35 to 1.17 ms. Chronaxie Values for human denervated skeletal muscles ranges from 9.5 to 30 ms-> this value drops again during reinnervation (see Geddes, L. A. (2004). Accuracy Limitations of Chronaxie Values. IEEE T Bio-Med Eng, 51(1), 176, to which the reader is referred for a fuller understanding).

The accommodation index is the ratio of threshold amperage of a slowly rising impulse to the threshold amperage of a suddenly rising rectangular impulse. Accommodation describes the response of excitable membranes to slow depolarizing currents without generation of an action potential. In nerve fibers, a slowly rising current up to double rheobase intensity inactivates sodium conductance before the depolarization reaches the threshold, and therefore, no action potential is generated. This is named a high accommodation rate. In muscle fibers, sodium conductance is much less inactivated by slowly rising currents, and therefore, an action potential is generated also with slowly rising currents of less than twice the rheobase intensity. This is named a low accommodation rate. The difference between the high accommodation rate of normal nerve fibers and the low accommodation rate of normal muscle fibers is the physiologic basis of the accommodation index. Denervated muscle fibers have the same or even a lower accommodation rate than normal muscle fibers (See Schuhfried O, Vacariu G, Paternostro-Sluga T: Reliability of chronaxie and accommodation index in the diagnosis of muscle denervation. Phys. Medizin Rehabil. Kurortmedizin 2005, 15:174-178, to which the reader is referred for a fuller understanding).

### Accomodation index for healthy persons:

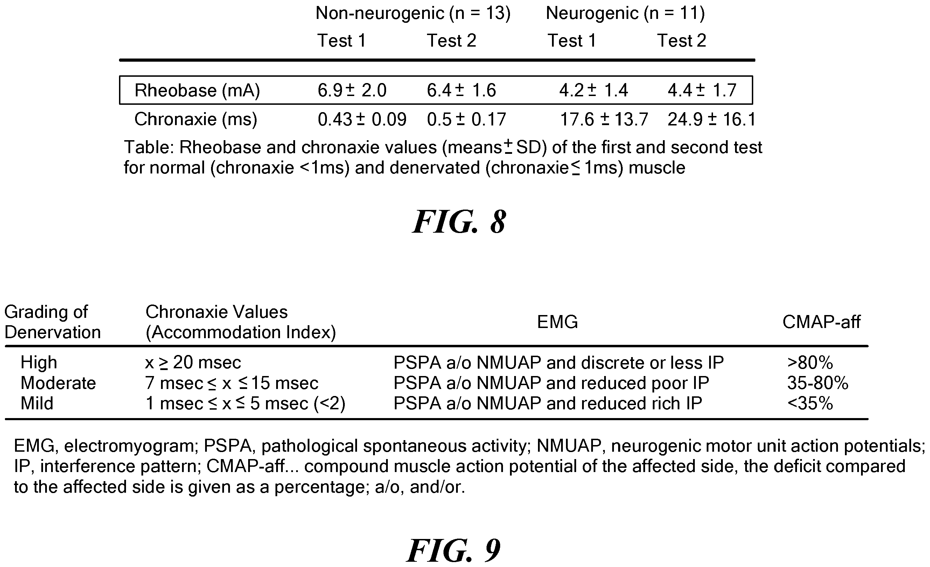

As described in Schuhfried et al., Fig. 8 shows different accommodation indices in human muscle groups consisting of the deltoid muscle, extensor digitorum communis muscle, the hypothenar muscle, the thibialis anterior muscle, the peroneus longus muscle and gastrochemius muscle. By applying rectangular and triangular impulses of 500 ms duration the accommodation index should be ≥2 in a healthy muscle.

### Accomodation index for persons with neurogenic lesion:

. Paternostro-Sluga, T., Schuhfried, O., Vacariu, G., Lang, T., & Fialka-Moser, V. (2002). Chronaxie and accommodation index in the diagnosis of muscle denervation. American Journal of Physical Medicine & Rehabilitation / Association of Academic Physiatrists, 81(4), 253-60. http://doi.org/10.1055/s-2004-834713 examined accommodation in human muscle group of 17 different muscles divided into three types: muscles of the upper extremity without the intrinsic muscles of the hand, intrinsic muscles of the hand, and muscles of the lower extremity and determined the accommodation index by dividing the threshold amperage of 500 ms duration by the rheobase values. As described in Paternostro-Sluga et al., Fig. 9 shows grading of muscle denervation according to electrical muscle testing and EMG. Accommodation indices (shown below) <2 indicated a neurogenic lesion.

### Accomodation index for persons with denervated muscles:

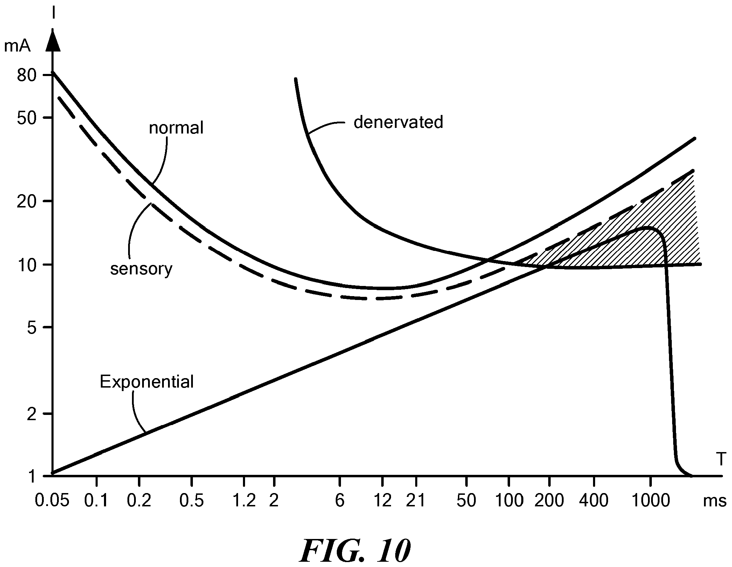

Cummings, J. P. (1985: The Journal of Orthopaedic and Sports Physical Therapy, 7(1), 11-5. Retrieved from http://www.ncbi.nlm.nih.gov/pubmed/18802291), to which the reader is referred for a fuller understanding, utilized selective electrical stimulation of denervated muscle with exponentially progressive current forms. Fig. 10 shows the principle of selective stimulation of denervated muscle utilizing a long exponentially progressive current form, as described in Cummings. The strength duration curves for normally innervated muscle, denervated muscle, and intact sensory nerves are illustrated. Selective stimulation of the denervated muscle without recruitment of either normally innervated muscle or the sensory axons is possible (shaded area). Following guidelines according to Thom H. Electrotherapy of paralysis; basic principles and application. [Internet]. Zeitschrift für Orthopädie und ihre Grenzgebiete 1953, 84: 104-23., to which the reader is referred for a fuller understanding, Cummins et al. showed that completely paralyzed muscle with advanced denervation atrophy will respond best to slowly rising impulses of 150 to 600 ms duration like shown in the shaded area in Fig. 10.

Thus, by using very long phase duration (>100ms) and slow rising pulse phase shapes, one expected to: 1. avoid stimulation of the underlying innervated muscles by increasing the thresholds for depolarization of motoneurons; and 2. avoid stimulation of the underlying nerves by increasing the thresholds for depolarization of excitable nerve membranes to slow depolarizing currents via surface electrodes, thereby selectively activating deep muscles showing signs of atrophy or denervation and accordingly showing excitable membranes to such slow depolarizing currents.

However, as described above, in illustrative embodiments of the invention these very slow rising, very long pulse phases surprisingly, reliably and selectively elicited full swallowing reflexes in human subjects. Not only were single muscles in the deep neck region activated, but full swallowing cycles were elicited that required coordinated contraction of all twelve or more muscle pairs with appropriate timing and peristaltic manner.

More specifically, in accordance with various embodiments of the invention, using bipolar pulses having a very long first pulse phase duration, i.e. >100ms, and having a slow rising shape of this pulse phase, a full swallowing reflex is elicited. Additionally, use of these bipolar pulses increased the thresholds for depolarization of excitable membranes and reduced depolarizing currents, thereby advantageously suppressing/avoiding the generation of action potentials in the motoneurons and their innervated muscles.. At the same time, the generation of action potentials in the mucosal pain fibers are also suppressed/avoided, thereby avoiding reaching the pain threshold.

In various embodiments of the invention, the magnitude of the first pulse phase of the bipolar stimulation pulse is increasing over time. For example, the first pulse phase of the bipolar stimulation signal may be progressively rising, such that it does not decrease in magnitude over time. In further embodiments, the first pulse phase of the bipolar stimulation signal may be exponentially rising, linearly rising, curvilinearly rising, continuously rising and/or discontinuously rising.

In the invention, a unipolar electrode configuration may be used, as described above, and unipolar stimulation pulses may be generated/delivered to elicit the full swallowing reflex. The unipolar stimulation pulse may be, without limitation, a triangular pulse. The unipolar stimulation pulse, such as the triangular pulse, has a rising edge with a duration of greater 100ms. In a unipolar electrode configuration, the anodic and cathodic pulse may depolarize different nervous (muscular) structures. The generated electrical field (e.g., using a triangular pulse) may advantageously depolarize the vocalis muscle more selectively. In various tests on healthy subjects, comparison between cathodic and anodic stimulation polarity (referring to the laryngeal electrodes) showed a lower activation threshold for the cathodic stimulation. The activation threshold was approximately two-fold higher for the anodic stimulation polarity. The swallowing reflex was elicited with both pulse polarities.

In accordance with illustrative embodiments of the invention, the system described herein may include implantable electrodes instead of surface electrodes as described herein so far. The implantable electrodes may be placed subcutaneous and attached e.g. to thyroid cartilage. An implantable controller may be configured to generate a stimulation signal for eliciting the full swallowing reflex in the human subject and configured to provide the stimulation signal to the plurality of implantable electrodes upon receipt of a trigger signal.

In accordance with illustrative embodiments of the invention, the system described herein may be used either as a rehabilitation or as a treatment system. The primary objective of rehabilitation is the restoration of disturbed functions by, for example, sensory stimulation of the swallowing reflex or teaching of special swallowing techniques. Necessary conditions for success are sufficient cortical potential after the injury and an existing connection from the cortex to the muscles. If this connection is lost or if the muscles cannot be sufficiently controlled, a rehabilitation of the swallowing process is not possible. Then, the patient is dependent on a diet via a feeding tube and a tracheal cannula.

In these cases, electrical stimulation of the external laryngeal muscles via activation of the swallowing reflex as a therapeutic approach may be used to enhance the swallowing process. Stimulation may have to be applied in a timely manner. Stimulation may be triggered either by the patient himself via a switch, such as a hand switch, or by using sensors as a trigger - for example, but not limiting, the electromyography (EMG) of the submental muscles or to measure bioimpedances (BI) at the neck (see, for example, www.mpi-magdeburg.mpg.de/bioimpedance, to which the reader is referred for a fuller understanding). Aspiration detection may be acquired via electrodes on the thyroid cartilage at the level of the vocal cords. The trigger signal generated by the switch or various sensors may be provided to the controller to initiate the generation and delivery of the stimulation signals that elicit the full swallowing response.

In accordance with further embodiments of the invention, the system may be used as assisting training of the patient via enhancing the exercises by stimulated swallowing - triggered, as described above, either by the patient himself via a hand-switch or by using sensors as trigger.

Although various exemplary embodiments of the invention have been disclosed, it should be apparent to those skilled in the art that various changes and modifications can be made which will achieve some of the advantages of the invention without departing from the true scope of the invention.

## Claims

1. A system for eliciting a full swallowing reflex in a human subject, the system comprising:
a plurality of surface electrodes configured to overlay at least thyroid cartilage in the neck of the human subject; and
a controller configured to:
generate a stimulation signal for eliciting the full swallowing reflex in the human subject, **characterised in that** the full swallowing reflex includes raising the larynx, the stimulation signals including one of:
a bipolar stimulation pulse having a first pulse phase duration of at least 100ms, and
a unipolar stimulation pulse that includes a rising edge with a duration of at least 100ms; and
provide the stimulation signal to the plurality of surface electrodes upon receipt of a trigger signal.

2. The system according to claim 1, wherein the first phase of the bipolar stimulation pulse has an increasing magnitude over time.

3. The system according to claim 2, wherein the first pulse phase of the bipolar stimulation signal is one of progressively rising, exponentially rising, linearly rising, curvilinearly rising, continuously rising or discontinuously rising, and combinations thereof.

4. The system according to claim 1, wherein the stimulation signals include a bipolar stimulation pulse, and wherein the surface electrodes include a first surface electrode and a second surface electrode in a bipolar configuration, the first surface electrode and the second surface electrode for positioning on the skin to the left and right of the laryngeal prominence, respectively, so as to overlay the region of thyroid cartilage in the neck.

5. The system according to claim 1, wherein the unipolar stimulation pulse is a triangular pulse that includes a rising edge with a duration of at least 100ms.

6. The system according to claim 1, wherein the surface electrodes include:
a first surface electrode and a second surface electrode for positioning on the skin to the left and right of the laryngeal prominence, respectively, so as to overlay the region of thyroid cartilage in the neck, and
a third surface electrode and a fourth surface electrode for placing on left and right shoulder blades of the human subject.

7. The system according to claim 1, wherein the full swallowing reflex includes, at least in part, raising the larynx.

8. The system according to claim 1, further comprising a trigger mechanism configured to provide the trigger signal.

9. The system according to claim 8, wherein the trigger mechanism includes one of a hand switch for activating by the human subject, a movement sensor, an inertial mass sensor, a sensor for electromyography of the submental muscles, or a sensor for measuring bioimpedances of the neck, and combinations thereof.

10. A system for eliciting a full swallowing reflex in a human subject, the system comprising:
a plurality of electrodes configured to be implanted adjacent at least thyroid cartilage in the neck of the human subject; and
a controller configured to:
generate a stimulation signal for eliciting the full swallowing reflex in the human subject, **characterised in that** the full swallowing reflex includes raising the larynx, the stimulation signals including one of:
a bipolar stimulation pulse having a first pulse phase duration of at least 100ms, and
a unipolar stimulation pulse that includes a rising edge with a duration of at least 100ms; and
provide the stimulation signal to the plurality of electrodes upon receipt of a trigger signal.

## Patentansprüche

1. System zum Auslösen eines vollständigen Schluckreflexes bei einem menschlichen Subjekt, wobei das System umfasst:
mehrere Oberflächenelektroden, die dafür ausgelegt sind, wenigstens den Schildknorpel im Hals des menschlichen Subjekts zu überlagern; und
eine Steuerung, die dafür ausgelegt ist:
ein Stimulationssignal zu erzeugen, um bei dem menschlichen Subjekt den vollständigen Schluckreflex auszulösen, **dadurch gekennzeichnet, dass** der vollständige Schluckreflex das Anheben des Kehlkopfes einschließt, wobei die Stimulationssignale eines der folgenden beinhalten:
einen bipolaren Stimulationsimpuls mit einer ersten Impulsphasendauer von wenigstens 100 ms und
einen unipolaren Stimulationsimpuls, der eine Anstiegsflanke mit einer Dauer von wenigstens 100 ms aufweist; und
das Stimulationssignal bei Empfang eines Auslösesignals an die mehreren Oberflächenelektroden bereitzustellen.

2. System gemäß Anspruch 1, wobei die erste Phase des bipolaren Stimulationsimpulses eine mit der Zeit zunehmende Stärke aufweist.

3. System gemäß Anspruch 2, wobei die erste Impulsphase des bipolaren Stimulationssignals entweder progressiv ansteigend, exponentiell ansteigend, linear ansteigend, kurvenförmig ansteigend, kontinuierlich ansteigend und/oder diskontinuierlich ansteigend und Kombinationen davon ist.

4. System gemäß Anspruch 1, wobei die Stimulationssignale einen bipolaren Stimulationsimpuls beinhalten und wobei die Oberflächenelektroden eine erste Oberflächenelektrode und eine zweite Oberflächenelektrode in einer bipolaren Konfiguration beinhalten, wobei die erste Oberflächenelektrode und die zweite Oberflächenelektrode zur Positionierung auf der Haut links bzw. rechts des Kehlkopfvorsprungs vorgesehen sind, um den Bereich des Schildknorpels im Hals zu überdecken.

5. System gemäß Anspruch 1, wobei der unipolare Stimulationsimpuls ein Dreiecksimpuls ist, der eine Anstiegsflanke mit einer Dauer von wenigstens 100 ms aufweist.

6. System gemäß Anspruch 1, wobei die Oberflächenelektroden beinhalten:
eine erste Oberflächenelektrode und eine zweite Oberflächenelektrode zur Positionierung auf der Haut links bzw. rechts des Kehlkopfvorsprungs, um den Bereich des Schildknorpels im Hals zu überdecken, und
eine dritte Oberflächenelektrode und eine vierte Oberflächenelektrode zur Platzierung auf dem linken und dem rechten Schulterblatt des menschlichen Subjekts.

7. System gemäß Anspruch 1, wobei der vollständige Schluckreflex wenigstens teilweise das Anheben des Kehlkopfes einschließt.

8. System gemäß Anspruch 1, ferner einen Auslösemechanismus umfassend, der dafür ausgelegt ist, das Auslösesignal bereitzustellen.

9. System gemäß Anspruch 8, wobei der Auslösemechanismus entweder einen Handschalter zur Aktivierung durch das menschliche Subjekt, einen Bewegungssensor, einen Trägheitssensor, einen Sensor für die Elektromyographie der submentalen Muskeln oder einen Sensor zur Messung der Bioimpedanzen des Halses oder Kombinationen davon umfasst.

10. System zum Auslösen eines vollständigen Schluckreflexes bei einem menschlichen Subjekt, wobei das System umfasst:
mehrere Elektroden, die dafür ausgelegt sind, benachbart zu wenigstens einem Schildknorpel im Hals des menschlichen Subjekts implantiert zu werden; und
eine Steuerung, die dafür ausgelegt ist:
ein Stimulationssignal zu erzeugen, um bei dem menschlichen Subjekt den vollständigen Schluckreflex auszulösen, **dadurch gekennzeichnet, dass** der vollständige Schluckreflex das Anheben des Kehlkopfes einschließt, wobei die Stimulationssignale eines der folgenden beinhalten:
einen bipolaren Stimulationsimpuls mit einer ersten Impulsphasendauer von wenigstens 100 ms und
einen unipolaren Stimulationsimpuls, der eine Anstiegsflanke mit einer Dauer von wenigstens 100 ms aufweist; und
das Stimulationssignal bei Empfang eines Auslösesignals an die mehreren Elektroden bereitzustellen.

## Revendications

1. Système permettant de déclencher un réflexe de déglutition complète chez un sujet humain, le système comprenant :
une pluralité d'électrodes de surface configurées pour recouvrir au moins le cartilage thyroïde dans le cou du sujet humain ; et
un dispositif de commande configuré pour :
générer un signal de stimulation pour déclencher le réflexe de déglutition complète chez le sujet humain, **caractérisé en ce que** le réflexe de déglutition complète comprend l'élévation du larynx, les signaux de stimulation comprenant l'un parmi :
une impulsion de stimulation bipolaire dont la durée de la première phase d'impulsion est d'au moins 100 ms, et
une impulsion de stimulation unipolaire comprenant un bord montant d'une durée d'au moins 100 ms ; et
fournir le signal de stimulation à la pluralité d'électrodes de surface lors de la réception d'un signal de déclenchement.

2. Système selon la revendication 1, la première phase de l'impulsion de stimulation bipolaire ayant une amplitude croissante dans le temps.

3. Système selon la revendication 2, la première phase d'impulsion du signal de stimulation bipolaire étant l'une parmi une augmentation progressive, une augmentation exponentielle, une augmentation linéaire, une augmentation curviligne, une augmentation continue ou une augmentation discontinue, et la combinaison de celles-ci.

4. Système selon la revendication 1, les signaux de stimulation comprenant une impulsion de stimulation bipolaire, et les électrodes de surface comprenant une première électrode de surface et une deuxième électrode de surface dans une configuration bipolaire, la première électrode de surface et la deuxième électrode de surface étant positionnées sur la peau à gauche et à droite de la proéminence laryngée, respectivement, de manière à recouvrir la région de cartilage thyroïde dans le cou.

5. Système selon la revendication 1, l'impulsion de stimulation unipolaire étant une impulsion triangulaire qui comprend un bord ascendant d'une durée d'au moins 100 ms.

6. Système selon la revendication 1, les électrodes de surface comprenant :
une première électrode de surface et une deuxième électrode de surface à positionner sur la peau à gauche et à droite de la proéminence laryngée, respectivement, de manière à recouvrir la région de cartilage thyroïde dans le cou, et
une troisième électrode de surface et une quatrième électrode de surface destinées à être placées sur les omoplates gauche et droite du sujet humain.

7. Système selon la revendication 1, le réflexe de déglutition complète comprenant, au moins en partie, l'élévation du larynx.

8. Système selon la revendication 1, comprenant en outre un mécanisme de déclenchement configuré pour fournir le signal de déclenchement.

9. Système selon la revendication 8, le mécanisme de déclenchement comprenant un élément parmi un interrupteur manuel à activer par le sujet humain, un capteur de mouvement, un capteur de masse inertielle, un capteur d'électromyographie des muscles sous-mentonniers, ou un capteur de mesure de bioimpédances du cou, et des combinaisons de ceux-ci.

10. Système permettant de déclencher un réflexe de déglutition complète chez un sujet humain, comprenant :
une pluralité d'électrodes configurées pour être implantées à proximité d'au moins le cartilage thyroïde dans le cou du sujet humain ; et
un dispositif de commande configuré pour :
générer un signal de stimulation pour déclencher le réflexe de déglutition complète chez le sujet humain, **caractérisé en ce que** le réflexe de déglutition complète comprend l'élévation du larynx, les signaux de stimulation comprenant l'un parmi :
une impulsion de stimulation bipolaire dont la durée de la première phase d'impulsion est d'au moins 100 ms, et
une impulsion de stimulation unipolaire comprenant un front montant d'une durée d'au moins 100 ms ; et
fournir le signal de stimulation à la pluralité d'électrodes lors de la réception d'un signal de déclenchement.
